# EUROPEAN PATENT SPECIFICATION

(11) **EP 0 458 820 B1**
(45) Date of publication and mention of the grant of the patent: **22.12.1993**
(21) Application number: 90902789.8
(22) Date of filing: 15.02.1990
(51) Int. Cl.: A61F 13/15

(54) **A DIAPER AND A METHOD FOR ITS PRODUCTION**
BABYWINDEL UND VERFAHREN ZUR HERSTELLUNG
COUCHE ET PROCEDE DE PRODUCTION CORRESPONDANT

(30) Priority: 20.02.1989 FI 890815
(43) Date of publication of application: 04.12.1991
(73) Proprietor: KOLMI-SET OY, SF-05801 Hyvinkää (FI)
(72) Inventor: HEIKKILÄ, Jouko, SF-07830 Pukaro (FI); PIRKKANEN, Jouko, SF-05860 Hyvinkää (FI); KÄYHKÖ, Leena, SF-04200 Kerava (FI)
(74) Representative: Harrison, Philippa Dinah
(86) International application number: FI9000047
(87) International publication number: WO9009159

(56) References cited:
- EP-A- 0 194 453
- DE-A- 3 423 644

## Description

This invention relates to a diaper comprising at least one liquid-impermeable surface layer, at least one liquid-permeable surface layer, an elongated suction pad disposed between said surface layers, and elastic bands stretched on both sides of the suction pad, the elastic bands being fastened at least over a part of their length to either one of the surface layers and arranged to follow, over the whole length of the diaper, paths reversed with respect to the longitudinal axis of the diaper, the ends of the bands being positioned on the same line parallel with the longitudinal direction of the diaper. In the method of the invention, the surface layers are fed in web form and joined into an integral diaper web which is cut into separate diapers, the longitudinal direction of the diapers corresponding to the longitudinal direction of the webs.

SE Published Specification 446 939, for instance, discloses an incontinence diaper of the type described above. In this diaper the elastic bands follow reversed paths over the entire length of the diaper. These paths are also symmetrical with respect to the transverse central line of the diaper. In embodiments where the elastic bands go outside the suction pad up to the ends of the diaper, the bands are not supported in any way over their length, especially not at the ends of the diaper. The lack of support causes the ends of the diaper to be pressed together by the elastic bands, and in an attempt to alleviate this drawback, the bands have been stretched less within the area of the ends. In addition, the elastic bands go relatively close to the suction pad in the middle region of the diaper, wherefore the diapers of this published specification cannot provide the best possible fit and tightness. One further reason for this may be the symmetrical shape of the diaper.

The object of the present invention is to provide a new diaper which avoids the above drawbacks and provides the best possible fit and tightness. This is achieved by means of a diaper according to the invention which is characterized in that the elastic bands are substantially tangent to the suction pad at points within the area of one end of the suction pad and diverge from it substantially linearly at least up to points within the area of the other end of the suction pad. The method of producing a diaper of this kind is characterized in that the elastic bands are arranged to follow zigzag paths which are substantially tangent to the suction pad at points within the area of one end of the suction pad of each diaper and diverge from it substantially linearly at least up to points within the area of the other end of the suction pad.

The points at which the elastic bands are tangent to the suction pad are preferably positioned within the area of the front end of the diaper.

In the diaper of the invention the elastic bands are supported to one end of the suction pad, so that the elastic bands can be properly stretched within the middle region of the diaper, which is to be positioned between the legs of the user, thus raising the edges of the diaper upward and forming a cup-shaped structure which efficiently prevents diaper leakage. As the elastic bands are supported to the suction pad so that the bands can be stretched tight, it can be ensured that the edges of the diaper are positioned tightly around the thighs of the user.

In the following the diaper of the invention will be described in greater detail with reference to the attached drawings, wherein
Figure 1 shows a first embodiment of the diaper of the invention;
Figure 2 shows a second embodiment of the diaper of the invention;
Figure 3 is a cross-sectional view of the middle region of the diaper of Figure 2 along the line III-III;
Figure 4 shows a third embodiment of the diaper of the invention; and
Figure 5 shows a fourth embodiment of the diaper of the invention.

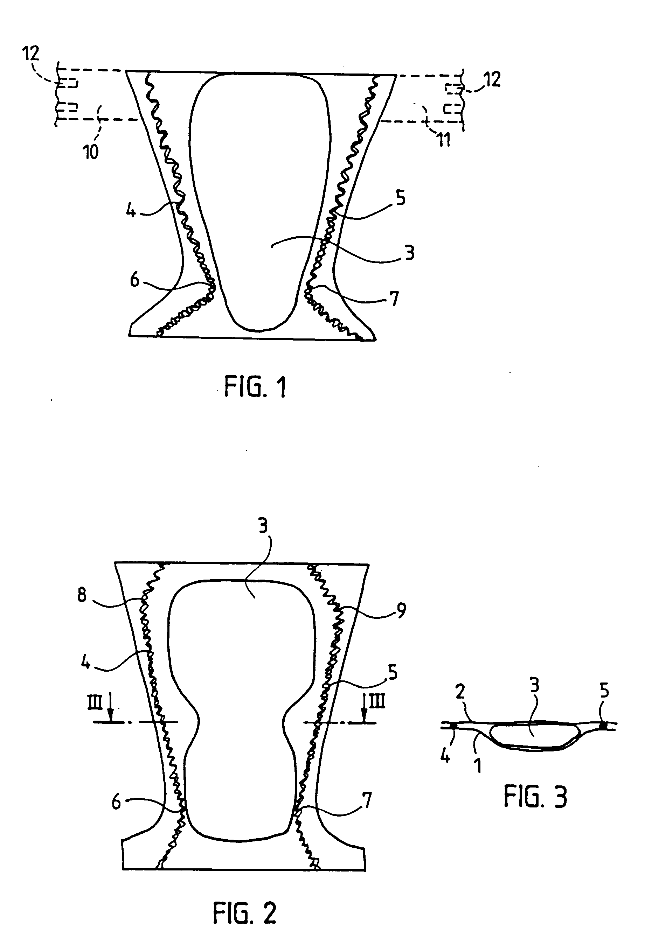

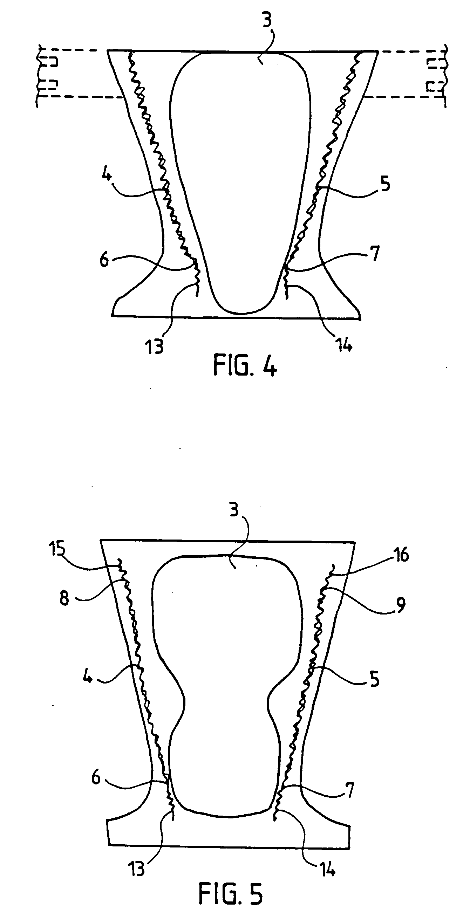

The diapers shown in Figures 1 to 5 comprise a first surface layer 1 of a material substantially impermeable to liquid, such as plastic, and a second liquid-permeable surface layer 2 to be positioned against the body of the user. An elongated suction pad 3 is fixed between the surface layers. The suction pad may consist of, for instance, cotton or cellulose crush and optionally comprises a region made of a superabsorbent material. Elastic bands 4 and 5 are provided on the sides of the diaper between the surface layers 1 and 2. They are arranged to follow paths reversed with respect to the longitudinal axis of the diaper so that their ends are positioned substantially on the same line in the longitudinal direction of the diaper. This is due to the fact that the diaper of the invention is made of a web formed by the surface layers, the individual diapers being cut off the diaper web. In the invention, the elastic bands 4 and 5, which may be formed by one or more separate bands which may be of different width, are disposed between the surface layers 1 and 2 so that they are substantially tangent to the suction pad 3 at points 6 and 7 within the area of one end of the suction pad 3 and diverge from the suction pad at least up to certain points within the area of the other end of the suction pad 3. In Figures 2 and 5, these points are indicated with the reference numerals 8 and 9. It is essential for the operation of the diaper of the invention that the elastic bands 4 and 5 are fixed in at least one of the surface layers 1 and 2 at least between the points 6 and 8 and the points 7 and 9, respectively. In the invention, the points 6 and 7, at which the elastic bands 4 and 5 are tangent to the suction pad 3, are positioned close to the front end of the diaper, that is, close to the end to the positioned at the front of the user.

In the embodiment shown in Figure 1, the elastic bands 4 and 5 are bent only at the points 6 and 7. The elastic bands 4 and 5 are attached to the diaper over their entire length. In the embodiments of Figure 1 and Figure 4, the suction pad 3 is substantially wedge-shaped. As clearly appears from the figures, the elastic bands 4 and 5 diverge from the suction pad 3 in the direction from the points 6 and 7 toward the other end of the suction pad, that is, toward its rear end. This divergence causes the bands 4 and 5 to raise the edges of the diaper upward when the diaper is bent in the middle into the shape in which it is to be used. Another factor contributing to the formation of the cup shape is that the elastic bands 4 and 5 are supported at the points 6 and 7 to the suction pad 3 having a considerably greater rigidity than the rest of the diaper. In this way the elastic bands 4 and 5 are not able to pull together the front portion of the diaper as the pad 3 is sufficiently rigid to compensate for the traction forces caused by them. An advantage of this is that the front portion of the diaper will not hang loosely when the diaper is in use. To illustrate the use of the diaper, Figures 1 and 4 show strips 10 and 11 indicated with broken lines. Each strip comprises at least one adhesive tape 12 by means of which the strips are to be fastened to the front portion of the diaper so that they will be positioned within the hip area of the user.

The embodiment of the diaper of the invention shown in Figure 2 differs from the embodiment shown in Figure 1 in that the suction pad 3 is therein somewhat hourglass-shaped and both elastic bands 4 and 5 comprise two bends. So the elastic bands follow a zigzag path over the length of each diaper. As far as the suction pad 3 is concerned, the embodiment of Figure 2 corresponds to that of Figure 5. In the embodiments of Figures 2 and 5, the suction pad generally widens toward its one end, that is, toward its rear end, so that the elastic bands 4 and 5 generally diverge from the suction pad 3 between the points 6 and 8 and the points 7 and 9, respectively. As a result, a similar cup shape is obtained as in the embodiments of Figures 1 and 4 for the same reasons. In the embodiment of Figure 2, the elastic bands 4 and 5 are also fixed in at least one of the surface layers 1 and 2 over their entire length.

The embodiment of Figure 4 deviates from that of Figure 1 in that the elastic bands 4 and 5 have not been glued in sections extending from the points 6 and 7 toward the front end of the diaper. In Figure 4, these sections 13 and 14 are shown as loosely curled band sections extending from points 6 and 7. In practice, the operation of the embodiment of Figure 4 does not differ from that of the embodiment of Figure 1 to any greater degree. So the band sections 13 and 14 are not of any greater importance for the operation of the diaper. At the production stage these sections 13 and 14 are passed similarly as in the embodiment of Figure 1 to achieve a web of elastic bands for the production of diapers by a web technique.

Figure 5 shows still another embodiment of the diaper of the invention, in which the band sections 13 and 14 extending from the points 6 and 7 toward the front portion of the diaper and band sections 15 and 16 extending from the points 8 and 9 toward the rear portion of the diaper are not fixed. The operation of the diaper of Figure 5 does not differ from that of the embodiment shown in Figure 2. The embodiment also comprises the important features of the invention, that is, the elastic bands 4 and 5 are tangent to the suction pad 3 at points within the front portion of the diaper and diverge from the pad in the direction toward the rear end of the diaper at least up to the points 8 and 9, which in the embodiment of Figure 5 are positioned relatively close to the rear edge of the suction pad 3, being fixed in at least either one of the surface layers between the points 6 and 8 and the points 7 and 9, respectively.

In the production of the diaper of the invention, the surface layers 1 and 2 are both fed in web form, the suction pads being attached by gluing, for instance, to the web 1, for instance, with suitable spacing. The elastic bands 4 and 5 are then fastened by gluing or hot sealing beside the suction pads according to the principles of the invention. Thereafter at least one liquid-permeable surface layer 2 is added to the diaper web. At this stage the web is cut into separate diapers. The required connecting strips can be attached either to the diaper web or to separated diapers by gluing, for instance. Alternatively, the diaper web can be formed by attaching the suction pad 3 and the elastic bands 4 and 5 to the liquid-permeable surface layer 2, the web forming the liquid-impermeable surface layer 1 being attached to the surface layer 2. Furthermore, it is possible to attach the suction pads 3 to one surface layer and the elastic bands 4 and 5 to the other surface layer, whereafter the webs so obtained are joined to form the finished diaper web.

The diaper of the invention and the method of producing such diapers has been described above by way of example by means of some specific embodiments, and it is to be understood that they can be modified in many ways without deviating from the basic idea of the invention and the scope of protection defined in the attached claims. Accordingly, the general shape of the diaper as well as that of the suction pad 3 can be altered without affecting the workability of the idea of the invention. The suction pad, for instance, can be fully rectangular or hourglass-shaped and symmetrical both in the longitudinal and transverse direction. Similarly, the entity formed by the surface layers may be symmetrical both in the longitudinal and transverse direction provided that the elastic bands can be positioned as required by the invention.

## Claims

1. A method of producing a diaper comprising at least one liquid-impermeable surface layer (1), at least one liquid-permeable surface layer (2), an elongated suction pad (3) disposed between said surface layers, and elastic bands (4, 5) stretched on both sides of the suction pad, the elastic bands (4, 5) being fastened at least over a part of their length to either one of the surface layers (1, 2) and arranged to follow, over the whole length of the diaper, paths reversed with respect to the longitudinal axis of the diaper, the ends of the bands being positioned on the same line parallel with the longitudinal direction of the diaper, wherein the surface layers (1, 2) are fed in web form and the elastic bands as continuous bands and joined with the suction pads to form an integral diaper web which is cut into separate diapers, the longitudinal direction of the diapers corresponding to the longitudinal direction of the webs, **characterized** in that the elastic bands (4, 5) are arranged to follow zigzag paths which are substantially tangent to the suction pad (3) at points (6, 7) within the area of one end of the suction pad (3) of each diaper and diverge from it substantially linearly at least up to points (8, 9) within the area of the other end of the suction pad.

2. A diaper comprising at least one liquid-impermeable surface layer (1), at least one liquid-permeable surface layer (2), an elongated suction pad (3) disposed between said surface layers, and elastic bands (4, 5) stretched on both sides of the suction pad (3), the elastic bands (4, 5) being fastened at least over a part of their length to either one of the surface layers (1, 2) and arranged to follow, over the whole length of the diaper, paths reversed with respect to the longitudinal axis of the diaper, the ends of the bands being positioned on the same line parallel with the longitudinal direction of the diaper, **characterized** in that the elastic bands (4, 5) are substantially tangent to the suction pad (3) at points (6, 7) within the area of one end of the suction pad (3) and diverge from it substantially linearly at least up to points (8, 9) within the area of the other end of the suction pad (3).

3. A diaper according to claim 2, **characterized** in that the points (6, 7) at which the elastic bands (4, 5) are tangent to the suction pad (3) are positioned within the area of the front end of the diaper.

## Patentansprüche

1. Verfahren zur Herstellung einer Windel, umfassend wenigstens eine flüssigkeitsundurchlässige Oberflächenschicht (1), wenigstens eine flüssigkeitsdurchlässige Oberflächenschicht (2), ein langgestrecktes Saugkissen (3), welches zwischen den Oberflächenschichten angeordnet ist, und elastische Bänder (4, 5), welche an beiden Seiten des Saugkissens gedehnt sind, wobei die elastischen Bänder (4, 5) wenigstens über einen Teil ihrer Länge an einer der Oberflächenschichten (1, 2) befestigt sind, und derart angeordnet sind, daß sie über die gesamte Länge der Windel Wegen folgen, welche bezüglich der Längsachse der Windel gebogen sind, wobei die Enden der Bänder auf der gleichen Linie parallel zur Längsrichtung der Windel angeordnet sind, worin die Oberflächenschichten (1, 2) in Gewebeform zugeführt werden und die elastischen Bänder als kontinuierliche Bänder und mit dem Saugkissen verbunden werden, um ein integrales Windelgewebe zu bilden, welches in einzelne Windeln geschnitten wird, wobei die Längsrichtung der Windeln der Längsrichtung der Gewebe entspricht,
**dadurch gekennzeichnet,**
daß die elastischen Bänder (4, 5) derart angeordnet sind, daß sie Zickzackwegen folgen, welche in Punkten (6, 7) im Bereich eines Endes des Saugkissens (3) jeder Windel im wesentlichen tangential zu dem Saugkissen (3) sind und von diesem im wesentlichen linear wenigstens bis zu Punkten (8, 9) im Bereich des anderen Endes des Saugkissens divergieren.

2. Windel, umfassend wenigstens eine flüssigkeitsundurchlässige Oberflächenschicht (1), wenigstens eine flüssigkeitsdurchlässige Oberflächenschicht (2), ein langgestrecktes Saugkissen (3), welches zwischen den Oberflächenschichten angeordnet ist, sowie elastische Bänder (4, 5), welche an beiden Seiten des Saugkissens (3) gedehnt sind, wobei die elastischen Bänder (4, 5) wenigstens über einen Teil ihrer Länge mit einer der beiden Oberflächenschichten (1, 2) verbunden sind und derart angeordnet sind, daß sie über die gesamte Länge der Windel Wegen folgen, welche bezüglich der Längsachse der Windel gebogen sind, wobei die Enden der Bänder auf der gleichen Linie parallel zur Längsrichtung der Windel angeordnet sind,
**dadurch gekennzeichnet,**
daß die elastischen Bänder (4, 5) in Punkten (6, 7) im Bereich eines Endes des Saugkissens (3) im wesentlichen tangential zum Saugkissen (3) sind und von diesem bis wenigstens zu Punkten (8, 9) im Bereich des anderen Endes des Saugkissens (3) im wesentlichen linear divergieren.

3. Windel nach Anspruch 2, **dadurch gekennzeichnet,** daß die Punkte (6, 7), an welchen die elastischen Bänder (4, 5) zum Saugkissen (3) tangential sind, im Bereich des vorderen Endes der Windel angeordnet sind.

## Revendications

1. Procédé de fabrication de couche-culotte comprenant au moins une couche de surface (1) imperméable au liquide, au moins une couche de surface (12) perméable au liquide, un coussin absorbant (3) allongé, placé entre lesdites couches de surface, et des rubans élastiques (4, 5) tendus sur les deux côtés du coussin absorbant, les rubans élastiques (4, 5) étant fixés sur une partie au moins de leur longueur à l'une ou l'autre des couches de surface (1, 2) et disposés pour suivre, sur toute la longueur de la couche-culotte, des trajets inversés par rapport à l'axe longitudinal de la couche-culotte, les extrémités des rubans étant placées sur une même ligne parallèle à la direction longitudinale de la couche-culotte, procédé dans lequel les couches de surface (1, 2) sont amenées sous forme d'une bande et les rubans élastiques sous forme de rubans continus et sont réunis aux coussins absorbants pour former une bande unique de couches-culottes que l'on découpe en couches-culottes séparées, la direction longitudinale des couches-culottes correspondant à la direction longitudinale des bandes, caractérisé en ce que les rubans élastiques (4, 5) sont disposés pour suivre des trajets en zigzag qui sont sensiblement tangents au coussin absorbant (3) en des points (6, 7) dans la région d'une extrémité du coussin absorbant (3) de chaque couche-culotte et qui en divergent de façon sensiblement rectiligne au moins jusqu'à des points (8, 9) situés dans la région de l'autre extrémité du coussin absorbant.

2. Couche-culotte comprenant au moins une couche de surface (1) imperméable au liquide, au moins une couche de surface (12) perméable au liquide, un coussin absorbant (3) allongé, placé entre lesdites couches de surface, et des rubans élastiques (4, 5) tendus sur les deux côtés du coussin absorbant (3), les rubans élastiques (4, 5) étant fixés sur une partie au moins de leur longueur à l'une ou l'autre des couches de surface (1, 2) et disposés pour suivre, sur toute la longueur de la couche-culotte, des trajets inversés par rapport à l'axe longitudinal de la couche-culotte, les extrémités des rubans étant positionnées sur la même ligne parallèle à la direction longitudinale de la couche-culotte, caractérisée en ce que les rubans élastiques (4, 5) sont sensiblement tangents au coussin absorbant (3) en des points (6, 7) dans la région d'une extrémité du coussin absorbant (3) et en divergent de façon sensiblement rectiligne au moins jusqu'à des points (8, 9) situés dans la région de l'autre extrémité du coussin absorbant.

3. Couche-culotte selon la revendication 2, caractérisée en ce que les points (6, 7) au niveau desquels les rubans élastiques (4, 5) sont tangents au coussin absorbant (3) sont placés dans la région de l'extrémité avant de la couche-culotte.
